# EUROPEAN PATENT SPECIFICATION

(11) **EP 3 573 716 B1**
(45) Date of publication and mention of the grant of the patent: **23.09.2026**
(21) Application number: 18702344.5
(22) Date of filing: 24.01.2018
(51) Int. Cl.: A61K 38/18, A61P 21/00

(54) **BIOLOGICALLY ACTIVE FUSION PEPTIDE FOR USE IN THE TREATMENT OF SPINAL MUSCULAR ATROPHY (SMA)**
BIOLOGISCH AKTIVES FUSIONSPEPTID ZUR VERWENDUNG IN DER BEHANDLUNG VON SPINALER MUSKELATROPHIE (SMA)
PEPTIDE DE FUSION BIOLOGIQUEMENT ACTIF DESTINÉ À ÊTRE UTILISÉ DANS LE TRAITEMENT DE L'AMYOTROPHIE SPINALE (SMA)

(30) Priority: 25.01.2017 IT 201700008082
(43) Date of publication of application: 04.12.2019
(73) Proprietor: Istituti Clinici Scientifici Maugeri Spa Società Benefit, 27100 Pavia (IT)
(72) Inventor: ROSSI, Daniela Maria Carmelita, 27100 Pavia (IT); BRAMBILLA, Liliana, 27100 Pavia (IT)
(74) Representative: Casci, Tamara
(86) International application number: PCT/IB2018/050425
(87) International publication number: WO 2018/138646

(56) References cited:
- WO-A1-2010/014746
- US-A1- 2009 156 789
- US-A1- 2012 309 687
- RYAN ANDERTON ET AL: "INVESTIGATION OF A RECOMBINANT SMN PROTEIN DELIVERY SYSTEM TO TREAT SPINAL MUSCULAR ATROPHY", TRANSLATIONAL NEUROSCIENCE, vol. 5, no. 1, 1 January 2014 (2014-01-01), XP055408197, DOI: 10.2478/s13380-014-0201-2
- ARTHUR H. M. BURGHES ET AL: "Spinal muscular atrophy: why do low levels of survival motor neuron protein make motor neurons sick?", NATURE REVIEWS. NEUROSCIENCE, vol. 10, no. 8, 8 July 2009 (2009-07-08), GB, pages 597 - 609, XP055408135, ISSN: 1471-003X, DOI: 10.1038/nrn2670
- FRANCESCA MARTORANA ET AL: "The BH4 domain of Bcl-XL rescues astrocyte degeneration in amyotrophic lateral sclerosis by modulating intracellular calcium signals", HUMAN MOLECULAR GENETICS, vol. 21, no. 4, 9 November 2011 (2011-11-09), gb, pages 826 - 840, XP055462115, ISSN: 0964-6906, DOI: 10.1093/hmg/ddr513

## Description

The present invention relates to a fusion peptide comprising an active peptide portion and a carrier peptide portion. The present invention further relates to the use of such peptide as medicament, particularly for use in the prevention, symptom postponement or treatment of Spinal Muscular Atrophy (SMA). The invention further relates to a pharmaceutical composition comprising said peptide and use thereof.

Spinal Muscular Atrophy (SMA) is a disorder of the nervous system characterized by the loss of α motor neurons in the spinal cord, resulting in proximal muscle weakness and paralysis. With an incidence of 1 in 6.000 to 10.000 live births and a carrier frequency of ~ 1:40, it is one of the most common autosomal recessive genetic disorders in humans. SMA is clinically classified into four phenotypes based on the age of onset and the severity of symptoms. These include a severe and infantile form (type I; Werdnig-Hoffmann disease), an intermediate form (type II), a "juvenile" form (type III; Kugelberg-Welander disease), and a form that presents with adult onset and mild course (type IV). The prognosis depends on the phenotypic severity, going from high mortality within the first year for SMA type I patients to no mortality for the chronic and later onset forms. Although the disease manifests with a wide spectrum of clinical gravity, it is clear that SMA represents the most frequent inherited cause of early mortality in childhood (Pearn J., Incidence, prevalence and gene frequency studies of chronic childhood spinal muscular atrophy, 1978, J. Med Genet 15:409-413 and Lefebvre et al, Identification and characterization of a spinal muscular athrophy-determining gene, 1995, Cell, 80:155-165*).* Besides, even patients with non-fatal forms of SMA are considerably disabled.

Contrary to other motor neuron disorders, SMA is a monogenic disease. Therefore, it is inherited according to mendelian principles, and sporadic cases are virtually unknown. Due to an intrachromosomal duplication event on chromosome 5, humans are the only species to carry two nearly identical copies of the disease-determining *"survival of motor neuron"* (*SMN*) gene, i.e. the telomeric *SMN1* and the centromeric *SMN2.* About 95% of patients have a homozygous disruption of *SMN1* due to deletion or gene conversion events, with the consequent reduction in the levels of its translated product, the SMN protein. Although patients retain a variable number of *SMN2* copies, a C->T transition in exon 7 causes aberrant splicing of 80-90% of SMN2-derived pre-mRNA transcripts. As a consequence, *SMN2* produces minor amounts of full-length transcripts and primarily generates transcripts lacking exon 7 (*SMN*Δ*7*), thus producing a protein with reduced stability. It follows that *SMN2* cannot fully compensate for the deficiency of *SMN1* and protect from SMA, though its copy number can influence the severity of the disease.

SMN is a widely and constitutively expressed protein, particularly abundant in motor neurons of the spinal cord. Although this protein has been implicated in a range of cellular processes, the precise pathway(s) linking SMN paucity to the pathogenesis of SMA remain(s) poorly understood.

The fact that the most severe and common form of SMA (Type I) manifests prenatally or in the early postnatal period, when the maturation of the neuromuscular system is still ongoing, suggests that disease processes may have their roots during the development. However, the onset of muscle weakness, in at least some cases of the later-onset forms of SMA, occurs after the neuromuscular system has stabilized, rather indicating that loss of neuromuscular integrity and progressive motor neuron degeneration are events of the post-developmental life.

In the recent years an approach based on the gene therapy against SMA has been studied, but unfortunately the obtained results showed some issues that make the delivery of genes into the nervous tissue still challenging, such as the large amount of virus required for the treatment as well as problems with immunogenicity in humans. Also manipulating *SMN2* pre-mRNA splicing by using antisense oligonucleotides (ASOs) have been investigated. Although splicing modification is an elegant therapeutic strategy, it has a limitation imposed by the amount of available *SMN2* pre-mRNAs. This is particularly disadvantageous for individuals affected by the most common and severe type I SMA, who have only one or two *SMN2* copies, but likely require the highest and most rapid induction of SMN. In addition, ASOs require intrathecal injections as they do not cross the intact blood-brain barrier when delivered systemically.

An alternative approach is that of conjugating a peptide with a suitable carrier. For instance, US 8,778,884 B2 discloses the use of the TAT-BH4 peptide for treating or preventing the progression of SLA (Amyotrophic Lateral Sclerosis), wherein the TAT domain is the protein transduction domain of the Human Immunodeficiency Virus (HIV) TAT protein, which is linked to the BH4 domain of Bcl-X*_{L}* through a (-GG-) linker.

Ryan et al. (January 2014) "Investigation of a recombinant SMN protein delivery system to treat spinal muscular atrophy", Translational Neuroscience, vol. 5, n.1, describes the attempts to use related carrier TAT peptides to deliver recombinant SMN protein to neurons.

US 2009/156789 A1 describes a fusion protein comprising a tag polypeptide and at least a portion of the SMN protein.

WO 2010/014746 A1 describes a fusion protein comprising the SMN polypeptide portion or fragment thereof, and a non-toxic botulinum neurotoxin (BoNT) portion or a fragment thereof as carrier in the prevention and treatment of SMA.

In view of the above, the Applicant has faced the problem to find a therapeutic approach against SMA, which also allows the prevention and symptom postponement of such disorder.

The Applicant has now solved the above-mentioned problems using a cell-penetrating peptide-based approach. In particular, the Applicant has now found a new fusion peptide comprising an active peptide portion and a carrier peptide portion, which represents a carrier systems enabling the entire peptide to cross the plasma membrane in neural cells as well as to pass through the blood-brain barrier (BBB).

In particular, the carrier system of the invention enables the peptide to cross plasma membrane in neural cells as well as to pass through the blood-brain barrier (BBB) after systemic administration.

Therefore, according to a first aspect, the present invention relates to a fusion peptide containing a carrier portion and an active peptide portion, said fusion peptide consisting of:
- a carrier peptide portion of SEQ ID NO: 1;
- an active peptide portion of SEQ ID NO: 28 (full-length SMN protein) or SEQ ID NO: 29 (i.e., the SMN²³⁵⁻²⁹⁴ carboxy-terminal domain, which is a fragment of the full-length SMN protein); and
- optionally a linking peptide sequence.

Described herein and not forming part of the invention is a fusion peptide comprising:
- a carrier peptide portion having an amino acid sequence selected from the group consisting of from SEQ ID NO: 2 to SEQ ID NO:27, preferably from SEQ ID NO: 2 to SEQ ID NO:6;
- an active peptide portion of SEQ ID NO: 28 (full-length SMN protein) or SEQ ID NO: 29 (i.e., the SMN²³⁵⁻²⁹⁴ carboxy-terminal domain, which is a fragment of the full-length SMN protein).

SEQ ID NO: 28 or SEQ ID NO: 29 are the full-length and a fragment, respectively, of the human SMN protein.

Advantageously, the fusion peptide of the invention represents a valid therapeutic approach against the Spinal Muscular Atrophy (SMA) as, once it is inside the cells, the biologically active portion can act in an appropriate and specific way by recognizing its intracellular molecular target. As for the penetrability of the fusion peptide into the cells, this is achievable through the carrier peptide portion coupled to the active peptide portion (which is a hydrophilic molecule), which increases the biomembrane permeability of such a hydrophilic molecule.

Furthermore, the fusion peptide of the invention showed to be effective in acting on neurite outgrowth and neuromuscular junctions (NMJs) formation. The peptide according to the present invention further showed to have a role in the prevention of neurite and cell degeneration. Therefore, the peptide of the invention is useful not only in the treatment of SMA, but also in the prevention of the same. Moreover the peptide of the invention is also useful in slowing the development of the disorder, as well as postponing symptom appearance and progression.

Another advantage lies in that the fusion peptide of the invention can be used in experimental models of SMA, in order to restore the physiological levels of SMN within the cells in the nervous tissue and beyond, to further test the efficacy of such a peptide and better understand the relevance of several events towards disease manifestation and progression, and then dissecting the biological processes controlled by SMA, as well as developing further novel therapeutic options.

### BRIEF DESCRIPTION OF THE DRAWINGS

Several embodiments of the present invention will now be described by way of example only, with reference to the accompanying drawings in which:
Figure 1 shows the determination of the expression levels of Smn mRNA and protein in the NSC-34 motor neural cells stably transfected with a shRNA construct against *Smn.*
Figure 2 shows the characterization of the morphological features of control and *Smn* knock-down motor neuronal cell lines.
Figure 3 shows the induction of cell death by Smn depletion in differentiated NSC-34 motor neuronal cells.
Figure 4 shows the effect of the peptides of the invention in promoting neuritogenesis and neurite outgrowth in the *Smn* knock-down NSC-34 motor neuronal cells.
Figure 5 shows the effect of the peptides of the invention in preventing apoptotic death of *Smn* knock-down NSC-34 motor neuronal cells.
Figure 6 shows the results of the analysis of the impact of the administration of a fusion peptide of the invention (TAT₄₈₋₅₇-flSMN) on motor function in SMN*Δ*7 (mouse model of SMA) compared to vehicle-treated mice. The tests performed were (A) Tail suspension test, (B) Negative Geotaxis and (C) Tube test.
Figure 7 is a photograph showing the appearance of a TAT₄₈₋₅₇-flSMN-treated SMN*Δ*7 mouse, a vehicle-treated SMN*Δ*7 mouse, a healthy wild-type (WT) mouse and a non-symptomatic heterozygous (HET) mouse at post-natal day (PND)12.

Further characteristics and advantages of the present invention will be evident from the following detailed description.

### DETAILED DESCRIPTION

For the scopes of the present invention, the terms and expressions reported below are to be intended as follows, unless otherwise specified.

The expression "fusion peptide" (or simply "peptide") of the invention means a peptide, oligopeptide, polypeptide or a protein obtained starting from the fusion of gene sequences, without any particular limiting reference to the length of the amino acid sequence.

The expression "active peptide portion" means a peptide having the full-length SMN protein sequence or its SMN²³⁵⁻²⁹⁴ carboxy-terminal domain which has an active role against SMA.

The expression "carrier peptide portion" means a carrier systems enabling the peptide to cross the plasma membrane in neural cells as well as to pass through the blood-brain barrier (BBB), also known as cell-penetrating peptide.

Specifically, the carrier peptide portion of the invention is the amino acid sequence of SEQ ID NO: 1. Specifically, described herein are carrier peptide portions selected from amino acid sequences of from SEQ ID NO: 1 to SEQ ID NO: 27. In particular, the amino acid sequences from SEQ ID NO: 1 to SEQ ID NO: 5 are protein transduction domains of the TAT protein from HIV virus, said domains being the domain TAT₄₈₋₅₇ (SEQ ID NO: 1), TAT₄₈₋₆₀ (SEQ ID NO: 2), TAT₄₉₋₅₇ (SEQ ID NO: 3), TAT₄₇₋₅₇ (SEQ ID NO: 4), TAT₄₇₋₅₆ (SEQ ID NO: 5), respectively. As for the other amino acid sequences from SEQ ID NO: 6 to SEQ ID NO: 27, they are peptide portions or protein transduction domains as indicated below. Particularly, such sequences are: PTD₄ (SEQ ID NO: 6) which is an optimized-version (artificial sequence) of TAT₄₇₋₅₇, Rn (polyarginines, R5-R12) (from SEQ ID NO: 7 to SEQ ID NO: 14) (i.e. repeated sequences of arginine (R)), pAntp(43-58) or penetratin (SEQ ID NO: 15) from Antennapedia homeodomain of *Drosophila melanogaster,* DPV1047 (SEQ ID NO: 16) which is an artificial sequence, pVEC (SEQ ID NO: 17) from murine vascular endothelial cadherin, ARF(1-22) (SEQ ID NO: 18) from human p14ARF protein, BPrPr(1-28) (SEQ ID NO: 19) from N-terminus of unprocessed bovine prion protein, MAP (SEQ ID NO: 20) which is an artificial sequence, transportan (SEQ ID NO: 21) from chimeric gelanin-mastoparan (mastoparan is from wasp (Vespula lewisii) venom), p28 (SEQ ID NO: 22) from bacterial azurin (Pseudomonas Aeruginosa), VT5 (SEQ ID NO: 23) which is an artificial sequence, Bac ₁₋₂₄ (SEQ ID NO: 24) from Bactenecin family of antimicrobial peptides from bovine neutrophils, C105Y (SEQ ID NO: 25) based on the amino acid sequence of human α1-Antitrypsin, PFVYLI (SEQ ID NO: 26) corresponding to a minimal active sequence of C105Y, Pep-7 (SEQ ID NO: 27) from CHL8 peptide phage clone, respectively.

According to the invention, the carrier peptide portion has amino acid sequence of SEQ ID NO: 1.

According to another aspect not according to the invention, the carrier peptide portion has an amino acid sequences selected from SEQ ID NO: 2, SEQ ID NO: 3, SEQ ID NO: 4, SEQ ID NO: 5 or SEQ ID NO: 6.

The peptide of the present invention can further comprise a linking peptide sequence placed between the peptide portion TAT and the active peptide portion. Such linking peptide can be selected in order to enable the adjacent portions (i.e. the active peptide portion and the carrier peptide portion) to move relative to one another or such that the inherent biological or therapeutic function of the active peptide portion and the carrier peptide portion must remain or improve or at least should and not be worsened after modification and conjugation. They are generally composed of small, nonpolar (e.g. Gly) or polar (e.g. Ser or Thr) amino acids to maintain flexibility, but can contain additional residues, such as Lys and Glu, to increase solubility. The length of the flexible linking peptides can be adjusted to allow for proper folding or to achieve optimal biological activity of the fusion peptides.

Preferably, the linking peptide has a length of 2 to 5 amino acid residues, more preferably all the amino acid residues being glycine (G) residues.

Preferably, the linking peptide has a length of 2 amino acid residues.

Preferably said linking peptide sequence consists of the amino acid residues (-GG-).

Without wishing to be bound by theory, it is believed that a small linking peptide, such as 2 to 5 amino acid residues long, is advantageous for sterical reasons, i.e. to ensure that two adjacent domains do not sterically interfere with one another. Moreover, a small linking peptide provides longer durability to the fusion peptide comprising it. The longer durability is ascribable to the fact that a small linking peptide is less susceptible than a larger one to undergoing chemical reactions in the surrounding environment and to proteolytic cleavages.

According to a preferred aspect, the fusion peptide of the invention consists of a carrier peptide portion of SEQ ID NO: 1, a linking peptide sequence, and an active peptide portion of SEQ ID NO: 28 or SEQ ID NO: 29. Preferably said linking peptide sequence consists of the amino acid residues (-GG-).

According to an aspect not according to the invention, the fusion peptide consists of a carrier peptide portion selected from the group consisting of from SEQ ID NO: 2 to SEQ ID NO: 27, more preferably SEQ ID NO: 2, SEQ ID NO: 3, SEQ ID NO: 4, SEQ ID NO: 5 or SEQ ID NO: 6, a linking peptide sequence, and an active peptide portion of SEQ ID NO: 28 or SEQ ID NO: 29. Preferably said linking peptide sequence consists of the amino acid residues (-GG-).According to another aspect, the present invention relates to the peptide as defined above for use as medicament.

According to another aspect, the present invention relates to the peptide as defined above for use in the prevention, symptom postponement or treatment of Spinal Muscular Atrophy (SMA).

In addition to the above-mentioned advantages related to the peptide of the invention, said peptide contributes to restore the physiological SMN levels within the cells, exerts axonotrophic activity and promotes the formation of myoneural synapses, as well as exerts anti-apoptotic effects *in vitro* and *in vivo.* Furthermore, the peptide of the present invention can promotes neurite formation and elongation in motor neural cells exhibiting reduced expression of the endogenous SMN protein. Moreover, the peptide of the invention can rescue the cells from the neurodegenerative process induced by SMN depletion.

As far as the administration routes are concerned, the peptide of the invention can be administered intraperitoneally or intravenously.

According to another aspect, the present invention relates to a combination of two fusion peptides, which comprises:
- a first fusion peptide containing a carrier portion and an active peptide portion, said fusion peptide consisting of:
   a) a carrier peptide portion of SEQ ID NO: 1;
   b) an active peptide portion of SEQ ID NO: 28 (full-length SMN protein); and
   optionally a linking peptide sequence; and
- a second fusion peptide containing a carrier portion and an active peptide portion, said fusion peptide consisting of:
   c) a carrier peptide portion of SEQ ID NO: 1;
   d) an active peptide portion of SEQ ID NO: 29 (i.e., the SMN²³⁵⁻²⁹⁴ carboxy-terminal domain, which is a fragment of the full-length SMN protein) and optionally a linking peptide sequence.

According to another aspect, not according to the invention, is described a combination of two fusion peptides, which comprises:
- a first fusion peptide comprising:
   a) a carrier peptide portion having an amino acid sequence selected from the group consisting of from SEQ ID NO: 2 to SEQ ID NO: 27, preferably SEQ ID NO: 2, SEQ ID NO: 3, SEQ ID NO: 4, SEQ ID NO: 5 or SEQ ID NO: 6; and
   b) an active peptide portion of SEQ ID NO: 28 (full-length SMN protein); and
- a second fusion peptide comprising:
   c) a carrier peptide portion having an amino acid sequence selected from the group consisting of from SEQ ID NO: 2 to SEQ ID NO: 27, preferably SEQ ID NO: 2, SEQ ID NO: 3, SEQ ID NO: 4, SEQ ID NO: 5 or SEQ ID NO: 6, and
   d) an active peptide portion of SEQ ID NO: 29 (i.e., the SMN²³⁵⁻²⁹⁴ carboxy-terminal domain, which is a fragment of the full-length SMN protein).

According to another aspect, the present invention relates to the combination of two peptides as defined above for use as medicament.

According to another aspect, the present invention relates to the combination of two peptides as defined above for use in the prevention, symptom postponement or treatment of Spinal Muscular Atrophy (SMA). Preferably the use of such combination is a simultaneous, separate or sequential use. Particularly, "simultaneous use" is understood as meaning the administration of the two fusion peptides according to the invention in a single pharmaceutical form. "Separate use" is understood as meaning the administration, at the same time, of the two fusion peptides according to the invention in distinct pharmaceutical forms. "Sequential use" is understood as meaning the successive administration of the two fusion peptides of the composition according to the invention, each in a distinct pharmaceutical form.

According to another aspect, the present invention relates to a pharmaceutical composition comprising the peptide or the combination of two fusion peptides as defined above. Preferably, such pharmaceutical composition comprises at least one pharmaceutically acceptable excipient.

According to another aspect, the present invention relates to a pharmaceutical composition as defined above for use in the prevention, symptom postponement or treatment of Spinal Muscular Atrophy (SMA).

The term "excipient" as used herein describes a material that does not cause significant irritation to an organism and does not abrogate the biological activity and properties of the active principle of the composition according to the present invention. Excipients must be of sufficiently high purity and of sufficiently low toxicity to render them suitable for administration to a subject being treated. The excipient can be inert, or it can possess pharmaceutical benefits.

According to a further aspect, the present invention relates to a pharmaceutical composition as defined above for use as medicament.

According to a further aspect, the present invention relates to a pharmaceutical composition as defined above for use in the prevention, symptom postponement or treatment of Spinal Muscular Atrophy (SMA) .

### EXAMPLES

The present invention will now be further illustrated by means of the following examples, which are illustrative only and are not intended to limit in any sense the scope of the invention.

### MATERIALS AND METHODS

### Cell line

As far as the cell line is concerned, the NSC-34 motor neural cell line was used. The NSC-34 cell line (Cashman et al. Neuroblastoma X Spinal Cord (NSC) hybrid cell lines resemble developing motor neurons, 1992, Developmental Dynamics 194:209-221*)* is a mouse-mouse hybrid cell line generated by fusion of neuroblastoma cells with motor neuron-enriched embryonic spinal cord cells. This cell line displays several phenotypic traits of motor neurons. NSC-34 cells are routinely maintained in Dulbecco's modified Eagle's medium (DMEM, Invitrogen), supplemented with 10% Foetal Bovine Serum (FBS, Gibco, Life Technologies), 2 mM glutamine, 1 mM Sodium Pyruvate and antibiotics (Euroclone).

The psiRNA-hH1neo G2 (InvivoGen) and sh_Smn_519 (Shafey et al. Hypomorphic Smn knockdown C2C12 myoblasts reveal intrinsic defects in myoblast fusion and myotube morphology, 2005, Exp Cell Res 311:49-61*)* constructs were transfected into the NSC-34 cells using FuGENE HD Transfection Reagent (Promega) according to the manufacturer's instructions. Forty-eight hours after the transfection, cells were subject to drug selection with 1 mg/ml G418 (Sigma-Aldrich) to obtain stably transfected cell lines. Two weeks after the transfection, single clones were picked and expanded in a DMEM/10% FBS/G418-based medium to obtain the psiRNA and sh_Smn_519 NSC-34 clonal cell lines, respectively. To induce differentiation, clones were maintained in culture with differentiation medium (DMed) (1:1 DMEM plus Ham's F12, 1% FBS, 1% penicillin/streptomycin, and 1% modified Eagle's medium nonessential amino acids; see 48).

### Real-Time Quantitative Reverse Transcription Polymerase Chain Reaction (RT-qPCR)

Total RNA was extracted from psiRNA and sh_Smn_519 NSC-34 clonal cell lines using RNeasy^{®} Mini kit (Qiagen) according to manufacturer's guidelines. 1 µg of cell extracted total RNA was reverse transcribed using iScript cDNA Synthesis kit (Bio-Rad Laboratories) according to manufacturer's instructions. The resulting cDNAs (2 ng) were analyzed by quantitative PCR using the SsoFast EvaGreen Supermix on a CFX96 real-time PCR Detection System (Bio-Rad Laboratories).

Relative expression of *Smn* was determined by the 2^{-ΔΔCt} method and normalized to hypoxanthine guanine phosphoribosyl transferase (*hprt*) gene expression. Transcripts were detected using the following primers: 5'-Smn-Tur (5'-CCAGAAGAAAACCTGCCAAGAA) (SEQ ID NO: 30) and 3'-Smn-Tur (5'-CACTTGTCACCAACTTTCCACTGT) (SEQ ID NO: 31); 5'-hprt (5'-TGAATCACGTTTGTGTCATTA) (SEQ ID NO: 32) and 3'-hprt (5'-TTCAACTTGCGCTCATCTTAG) (SEQ ID NO: 33).

### Western Blotting

Proteins were extracted from psiRNA and sh_Smn_519 NSC-34 clonal cell lines using RIPA lysis buffer (Pierce), supplemented with protease inhibitor cocktail (Complete Mini, Roche Diagnostics). Protein concentrations were measured using Pierce^{®} BCA Protein Assay Kit (Pierce). Proteins were electrophoretically separated onto a 12.5% SDS-polyacrylamide gel, and transferred onto a Nitrocellulose Transfer Membrane (Whatman). Smn was detected by incubation with mouse anti-Smn antibody (1:10000; BD Transduction Laboratories) followed by horseradish peroxidase-conjugated goat anti-mouse IgG (Sigma-Aldrich). Membranes were stripped and incubated with an anti-β-actin monoclonal antibody (1:5000; Sigma-Aldrich).

### Peptide characterization in vitro

Twenty-four hours after plating, psiRNA and sh_Smn_519 NSC-34 stable clones were treated with various concentrations of transducible fusion protein/peptide (3 hours). Protein/peptide in excess were subsequently removed and cells were maintained in culture in DMed for 24 and 48 hours. Cells were then fixed and stained, as described below. Neurite outgrowth and/or cell death were subsequently analyzed (see "Immunocytochemistry") .

Biologically active protein/peptidic moieties taken into consideration were: the full-length SMN or its SMN²³⁵⁻²⁹⁴ carboxy-terminal domain. Protein/peptide were synthesized by highly qualified and ISO 9001 certified companies, as previously described (Martorana et al, The BH4 domain of Bcl-X(L) rescues astrocyte degeneration in amyotrophic lateral sclerosis by modulating intracellular calcium signals, 2012, Hum Mol Genet 21:826-840*).*

### Immunocytochemistry

Cells were rinsed in phosphate-buffered saline (PBS) and fixed in 4% PFA for 20 min. To evaluate nuclear condensation, nuclei were stained with bis-Benzimide (Hoechst 33342, 10 µg /ml in PBS) for 15 minutes at room temperature, before fixing the cells. The number of cells showing condensed nuclei was counted as previously described.

For immunostaining, cells were incubated for 1 hour with a blocking/permeabilizing solution (5% Serum, 0.1% Triton X-100, in PBS) at room temperature. To evaluated neurite outgrowth, cells were immunostained using a mouse antibody to β-tubulin isotype III (Sigma-Aldrich). Neurite length was measured according to a protocol previously established in our laboratory using ImageJ software (National Institutes of Health) and NeuronJ plug-in. Briefly, only processes as long as twofold the average cell body dimension were considered neurites and measured. The following parameters were collected: percentage of cells without neurites (cells without neurites/total number of cells) and mean length of the longest neurite per cell (total length of the longest neurite of each cell with neurites/number of cells with neurites). A total of about 100 cells per group were analyzed. Data were presented as mean ± s.e.m. from at least three independent experiments.

### Pharmacological treatment in vivo

Animals (SMNΔ7 SMA mice) were treated with recombinant TAT₄₈₋₅₇-flSMN or the equivalent volume of vehicle solution according to the following administration protocol. An initial intravenous administration of 17 mg/kg TAT₄₈₋₅₇-flSMN or the equivalent volume of vehicle solution were performed into the mouse facial vein at post-natal day (PND)1, followed by an intraperitoneal administration of the same dose of protein or equivalent volume of vehicle solution at PND5.

### Behavioral tests

To evaluate the impact of TAT₄₈₋₅₇-flSMN on the onset and progression of the disease, vehicle- (20 mice) and TAT₄₈₋₅₇-flSMN-treated mice (19 mice) were subject to the following battery of behavioral tests, every second day, from post-natal day (PND)2 or PND4 to PND12:
- Tail suspension test (PND2-12): Mice were suspended by the tail for 15 seconds and their hind-limb posture scored: 4, normal, hind limbs spread open; 3, hind limbs not completely spread; 2, hind limbs often close together; 1, hind limbs always close together; 0, hind limbs always close together with postural abnormalities of the extremities (clasping).
- Negative geotaxis test (PND4-12): This test evaluates motor coordination and vestibular sensitivity. Negative geotaxis was tested by placing the mouse on an inclined grid (at approximately 35° inclination) with the mouse head facing down and recording for a maximum of 60 seconds the time it took for them to turn around and climb upwards. The mice were given a score as follows: 3, mice turn around and climb upwards within 60 seconds; 2, mice try but fail to turn around within 60 seconds; 1, mice fail to perform the test altogether, owing to a clear inability to stand as a result of significant muscle weakness and almost complete paralysis; 0, mice die before PND12.
- Tube test (PND2-12): This is a non-invasive motor function test specifically designed to evaluate proximal hind-limb muscle strength, weakness and fatigue in mouse neonates. In addition, it assesses general neuromuscular function, body muscle strength and posture. The test was performed in 2 consecutive trials per time-point on each mouse. In each trial, the mouse was placed head down, hanging by its hind limbs in a plastic 50 ml centrifuge tube with a cotton ball cushion at the bottom to protect the animal head upon its fall. Three parameters were evaluated:
   i) Time spent hanging onto the lip of the tube before falling down;
   ii) Number of pulls that the animal performs when attempting to escape the tube;
   iii) Hind-limb score (HLS), assessing the position of the legs and tail. The posture adopted was scored according to the following criteria: 4, normal, hindlimb separation with tail raised; 3, hind limbs closer together, but they rarely touch each other; 2, hind limbs close to each other, often touching; 1, hind limbs almost always in a clasp position with the tail raised; 0, constant clasping of the hind-limbs with the tail lowered. The HLS score is an overall evaluation of the hind-limb spread during the first 10-15 seconds of hanging onto the lip of the tube, after which the observer does not change the score even if the animal shows a lower or higher score.

Time spent hanging, number of pulls and hind-limb score were measured and the average of two measurements was inserted into the following equation to obtain a quantitative score: Tube Test Score (TTS) = [(time spent hanging) + 10(# of pulls)] x [(HLS score + 1)/4] for each time-point.

### Statistics

Data were represented as mean ± s.e.m. and statistical significance was verified using GraphPad Prism^{®} software. Unpaired 2-tailed t-test was used for comparisons between two groups; one-way analysis of variance (ANOVA) followed by Bonferroni *post-hoc* test was used for comparisons of multiple groups.

### EXAMPLE 1

### Isolation of a clone having low Smn expression levels

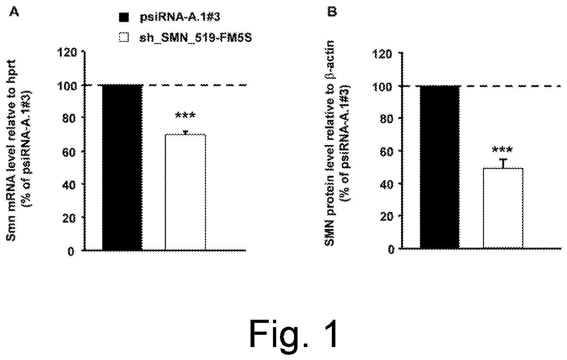

As first step, a cellular model of SMA by silencing the expression of the endogenous Smn protein in the NSC-34 motor neuronal cell culture system was generated. More specifically, NSC-34 motor neuronal cells were transfected with the psiRNA-hH1neo G2 control or the sh_Smn_519 silencing vectors (Shafey et al. Hypomorphic Smn knockdown C2C12 myoblasts reveal intrinsic defects in myoblast fusion and myotube morphology, 2005, Exp Cell Res 311:49-61*),* the latter being deputed to knock-down the expression of the endogenous *Smn* gene. Various stably transfected clones per each group were picked, expanded, and characterized for the levels of Smn expression by Western blotting (Figure 1). Among cells transfected with the psiRNA-hH1neo G2 control construct, two clones, named psiRNA-A.1#2 and psiRNA-A.1#3, were isolated. Since they showed similar morphology, proliferation rate and levels of Smn expression, the psiRNA-A.1#3 clonal cell line was chosen as representative of control conditions and used for subsequent experiments. The group transfected with the sh_Smn_519 plasmid exhibited problems of long-term maintenance after several passages, possibly because of the negative impact on cell survival of low Smn levels (see data below). To overcome this limitation, we isolated and characterized a large number of clones. Among these, one *Smn* knock-down clonal cell line, named sh_Smn_519-FM5S, was selected as representative of low Smn expression levels for further experiments.

With reference to **Figure** 1, it refers to the determination of the expression levels of Smn mRNA and protein in the NSC-34 motor neural cells stably transfected with a shRNA construct against *Smn.* Particularly, as for **Figure 1-A**, the total RNA was extracted from the NSC-34 clonal cell lines stably transfected with the psiRNA-hH1neo G2 control or the sh_Smn_519 silencing vectors, reverse transcribed and analyzed by RT-qPCR analysis. The psiRNA-A.1#3 cell line was used as representative of control conditions. Values (mean ± s.e.m.) were normalized relative to *hprt* and expressed as percentage of *Smn* mRNA levels in psiRNA-A.1#3 cell line. ***p<0.001 vs psiRNA-A.1#3, two-tailed unpaired t-test, n=12. As for **Figure 1-B**, lysates of the NSC-34 clonal cell lines stably transfected with the psiRNA-hH1neo G2 control or the sh_Smn_519 silencing vectors were analyzed by SDS-PAGE/Western blot using antibodies against mouse Smn and β-actin. Bands were densitometrically analyzed and Smn/β-actin ratios were calculated for normalization. The psiRNA-A.1#3 cell line was used as representative of control conditions. Sh_Smn_519-FM5S exhibits a 50% reduction in Smn expression levels when compared to the psiRNA-A.1#3 cells. Data (mean ± s.e.m.) are expressed as % of Smn expression levels in the psiRNA-A.1#3 cell line. ***p<0.001 vs psiRNA-A.1#3, two-tailed unpaired t-test, n= 3.

### EXAMPLE 2

### Impact of SMN depletion on several events

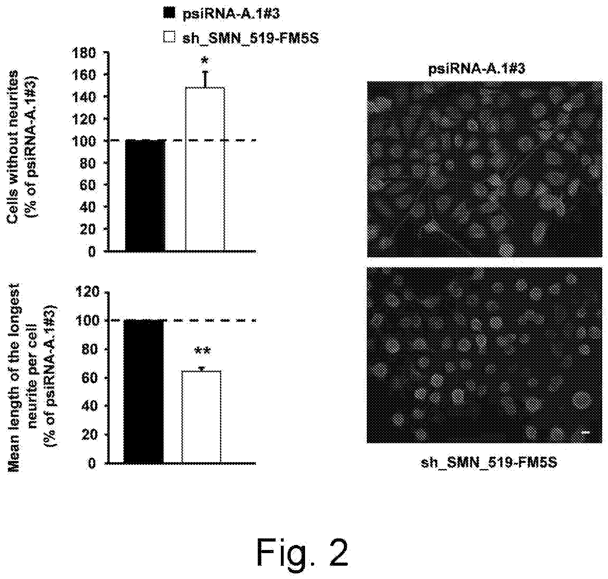

In order to study the impact of Smn depletion on neuritogenesis, neurite growth and cell death, the response of each clonal cell line to differentiating conditions (DMed: 1:1 DMEM plus Ham's F12, 1% FBS, 1% penicillin/streptomycin, and 1% modified Eagle's medium nonessential amino acids) was then determinated. Morphometric analysis indicated that, 48 hours after the switch to DMed, the sh_Smn_519-FM5S knock-down clonal cell line exhibited a significantly higher number of cells without neurites than the control psiRNA-A.1#3 clone (Figure 2). Noteworthy, this defect in neuritogenesis inversely correlated with the expression levels of the endogenous Smn protein (see Figure 1). In parallel, we identified a reduction in the mean length of the longest neurites per cell in the sh_Smn_519-FM5S clone (Figure 2), suggesting a defect in neurite outgrowth commitment that strictly correlated with the levels of Smn expression (see Figure 1).

With reference to **Figure 2****,** it refers to the characterization of the morphological features of control and *Smn* knock-down motor neuronal cell lines. Particularly, decreasing the expression of the Smn protein increases the number of cells without neurites (top) and reduces the mean length of the longest neurite per cell (bottom), 48 hours after the switch to differentiating conditions. Data (mean ± s.e.m.) are expressed as percentage of the psiRNA-A.1#3 control cell line (cells without neurites: 12.85 ± 1.25%; mean length of the longest neurite per cell: 103.9 ± 4.26 µm). *p<0.05 and **p<0.001 vs psiRNA-A.1#3, two-tailed unpaired t-test, n = 7 experiments in triplicate. Images are representative of the most significant effects. Scale bar, 20 µm.

### EXAMPLE 3

### Extent of apoptotic cell death

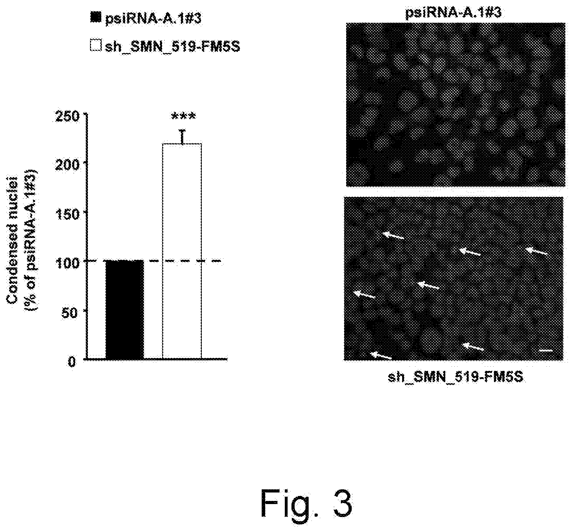

Subsequently, the extent of apoptotic cell death achieved under the same experimental conditions was investigated. The magnitude of motor neuron apoptosis was assessed by quantifying the number of NSC-34 cells with nuclear condensation. Differences in apoptotic parameters between the *Smn* knock-down cell line and the control cells became significant 48 hours after the switch to differentiating conditions (Figure 3). In addition, we found that the extent of cell death inversely correlated with the levels of Smn expression (see Figure 1).

Altogether, these results indicate that Smn depletion can influence neuritogenesis, neurite outgrowth and cell death in the NSC-34 motor neuronal cell culture system, thus posing the bases for testing the effects of TAT-flSMN and TAT-SMN²³⁵⁻²⁹⁴ on these cellular models of SMA.

With reference to **Figure 3****,** it refers to the induction of cell death by Smn depletion in differentiated NSC-34 motor neuronal cells. Particularly, the quantification of the number of cells with condensed nuclei reveals that *Smn* knock-down induces apoptotic cell death in the NSC-34 cells. Apoptosis inversely correlates with Smn expression levels in the different clonal cell lines (see Figure 1). The effect becomes evident at 48 hours after the switch to differentiating conditions (n = 17 experiments in duplicate). Data (mean ± s.e.m.) are expressed as percentage of condensed nuclei in the psiRNA-A.1#3 control cell line (0.44 ± 0.04%). ***p<0.0001 vs psiRNA-A.1#3, two-tailed unpaired t-test. Images are representative of conditions at 48 hours after the switch to differentiating medium. Arrows show condensed nuclei. Scale bar, 20 µm.

### EXAMPLE 4

### Axonotrophic and neuroprotective activity

In subsequent experiments, we therefore characterized *in vitro* the axonotrophic and neuroprotective activity of the TAT₄₈₋₅₇-flSMN fusion protein and TAT₄₈₋₅₇-SMN²³⁵⁻²⁹⁴ fusion peptide.

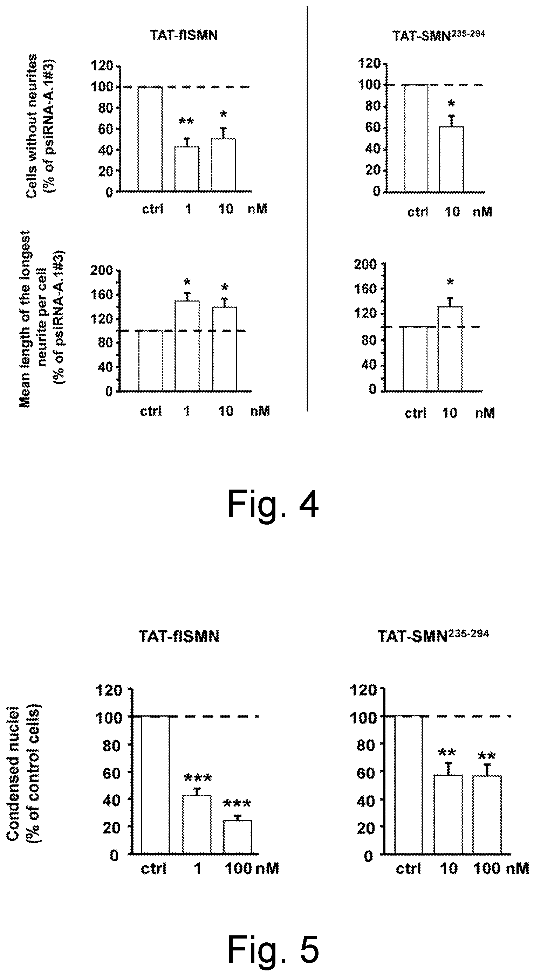

To this end, the sh_Smn_519-FM5S clone was initially pre-incubated for 3 hours with a range of concentrations of TAT₄₈₋₅₇-flSMN protein (1-100 nM), i.e. the peptide of the present invention comprising the peptide of sequence SEQ ID NO: 28 as active peptide portion, or TAT₄₈₋₅₇-SMN²³⁵⁻²⁹⁴ peptide (10-100 nM), i.e. the peptide of the present invention comprising the peptide of sequence SEQ ID NO: 29 as active peptide portion. Cells were then subjected to differentiating conditions and maintained in the presence of TAT₄₈₋₅₇-flSMN/ TAT₄₈₋₅₇-SMN²³⁵⁻²⁹⁴ for up to 48 hours. The impact of the two molecules in promoting the formation and elongation of neurites was assessed. Morphometric analyses revealed that both TAT₄₈₋₅₇-fusion peptides were effective in reducing the number of cells without neurites as well as in increasing the mean length of the longest neurite per cell (Figure 4). Yet, TAT₄₈₋₅₇-flSMN was already effective at 1 nM, while TAT₄₈₋₅₇-SMN²³⁵⁻²⁹⁴ exhibited significant effects only at concentrations above 10 nM (Figure 4). Interestingly, at the same concentration of 10 nM, TAT₄₈₋₅₇-flSMN and TAT₄₈₋₅₇-SMN²³⁵⁻²⁹⁴ significantly diminished the number of sh_Smn_519-FM5S cells without neurites of about 50% and 40%, respectively. Consistent with this, TAT₄₈₋₅₇-flSMN and TAT₄₈₋₅₇-SMN²³⁵⁻²⁹⁴ increased the mean length of the longest neurite per cell of 38% and 30%, respectively, in the sh_Smn_519-FM5S clone (Figure 4). On the whole, these results suggest that both molecules can promote the formation and elongation of neurites in the Smn deficient motor neuronal cells, though TAT₄₈₋₅₇-flSMN appears to be more effective than TAT₄₈₋₅₇-SMN²³⁵⁻²⁹⁴.

With reference to **Figure 4****,** it shows that the peptides TAT₄₈₋₅₇-flSMN and TAT₄₈₋₅₇-SMN²³⁵⁻²⁹⁴ promote neuritogenesis and neurite outgrowth in the *Smn* knock-down NSC-34 motor neuronal cells. Particularly, the sh_SMN_519-FM5S clonal cell line was pre-treated in the absence or in the presence of various concentrations of the TAT₄₈₋₅₇-flSMN protein or TAT₄₈₋₅₇-SMN²³⁵⁻²⁹⁴ peptide, before switching to differentiating conditions. Twenty-four hours after the switch to DMed, we detected a decrease in the number of cells without neurites and an increase in the mean length of the longest neurite per cell in the treated groups when compared to the untreated condition (ctrl). Data (mean ± s.e.m.) are expressed as percentage of untreated sh_SMN_519-FM5S cells (cells without neurites: sh_SMN_519-FM5S: 18.98 ± 1.74%; mean length of the longest neurite per cell: sh_SMN_519-FM5S: 66.54 ± 3.22 µm). *p<0.05 and **p<0.01 vs control cells, one-way ANOVA followed by Bonferroni post-hoc test, n = 3-4 experiments in triplicate.

### EXAMPLE 5

### Neuroprotective activity

Subsequently, the neuroprotective activity of the two peptides was evaluated. Sh_Smn_519 NSC-34 cells were subjected to the same experimental paradigm, as above. Forty-height hours after the switch to DMed, the impact of the two TAT₄₈₋₅₇-fusion peptides towards cell death was determined in terms of reduction in the number of cells with condensed nuclei. It was found that a concentration of TAT₄₈₋₅₇-flSMN and TAT₄₈₋₅₇-SMN²³⁵⁻²⁹⁴ as low as 100 nM was particularly effective in preventing sh_SMN_519-FM5S degeneration, although even lower concentrations resulted significantly protective towards apoptotic cell death (1-100 nM). More specifically, treating the sh_Smn_519-FM5S cells with 100 nM TAT₄₈₋₅₇-flSMN or TAT₄₈₋₅₇-SMN²³⁵⁻²⁹⁴ diminished the number of cells with condensed nuclei of about 75% and 45%, respectively (Figure 5).

With reference to **Figure** 5, it refers to the prevention activity of the peptides TAT₄₈₋₅₇-flSMN and TAT₄₈₋₅₇-SMN²³⁵⁻²⁹⁴ against apoptotic death of *Smn* knock-down NSC-34 motor neuronal cells. The sh_SMN_519-FM5S clonal cell line was pre-treated in the absence or in the presence of various concentrations of the TAT₄₈₋₅₇-flSMN peptide or the TAT₄₈₋₅₇-SMN²³⁵⁻²⁹⁴ peptide, before switching to differentiating conditions. Forty-eight hours after the switch to DMed, the number of cells with condensed nuclei decreased when compared to untreated cells (ctrl). Data (mean ± s.e.m.) are expressed as percentage of condensed nuclei in the untreated sh_SMN_519-FM5S cells (sh_SMN_519-FM5S: 0.98 ± 0.07%). **p<0.001 and ***p<0.0001 vs control cells, one-way ANOVA followed by Bonferroni post-hoc test, n = 5-7 experiments in triplicate.

### EXAMPLE 6

### Efficacy of the TAT₄₈₋₅₇-flSMN treatment in vivo, in SMNΔ7 SMA mice

Based on the results obtained with the experiments *in vitro,* it was decided to evaluate the therapeutic efficacy of TAT₄₈₋₅₇-flSMN *in vivo,* in the SMN*Δ*7 mouse model of SMA. This mouse line was generated by introduction into mice knocked-out for the endogenous *Smn* gene of two human SMN2 and SMN*Δ*7 transgenes (Le et al. SMNDelta7, the major product of the centromeric survival motor neuron (SMN2) gene, extends survival in mice with spinal muscular atrophy and associates with full-length SMN, Hum Mol Genet, 2005, 14:845-857*).* The SMN*Δ*7 mouse line was specifically selected because, in these animals, the disease is modulated by human SMN, in line with the nature of the TAT₄₈₋₅₇-fusion peptides of the invention.

*Ad hoc* breeding pairs were set up in order to obtain different cohorts of SMN*Δ*7 mice. The breeding scheme implies the concomitant generation of transgenic littermates for SMN2 and SMN*Δ*7 that carry two *Smn* knock-out alleles (SMNΔ7 mice), or one *Smn* knock-out and one *Smn* wild-type allele (heterouzygous, HET), or two *Smn* wild-type alleles (wild-type, WT). SMN*Δ*7 animals were subsequently treated with recombinant TAT₄₈₋₅₇-flSMN (19 mice) or vehicle solution (20 mice) according to the following administration protocol. 17 mg/kg TAT₄₈₋₅₇-flSMN protein or the equivalent volume of vehicle solution were initially administered intravenously into the mouse facial vein at post-natal day (PND)1. This was followed by a second intraperitoneal administration of the same dose of protein or equivalent volume of vehicle solution at PND5.

To evaluate the impact of the molecule on the onset and progression of the disease, vehicle- and TAT₄₈₋₅₇-flSMN-treated mice were subjected to a battery of behavioral tests (see the above section "*Behavioral tests*"), every second day, from PND2 (or PND4) to PND12. To optimize reproducibility, all tests were performed at the same time of the day and examiners were unaware of the mouse treatment during measurements.

More specifically, the battery of tests used in this project included:
- Tail Suspension test (one trial per animal per time point);
- Negative Geotaxis test (one trial per animal per time point);
- Tube test (two trials per animal per time point).

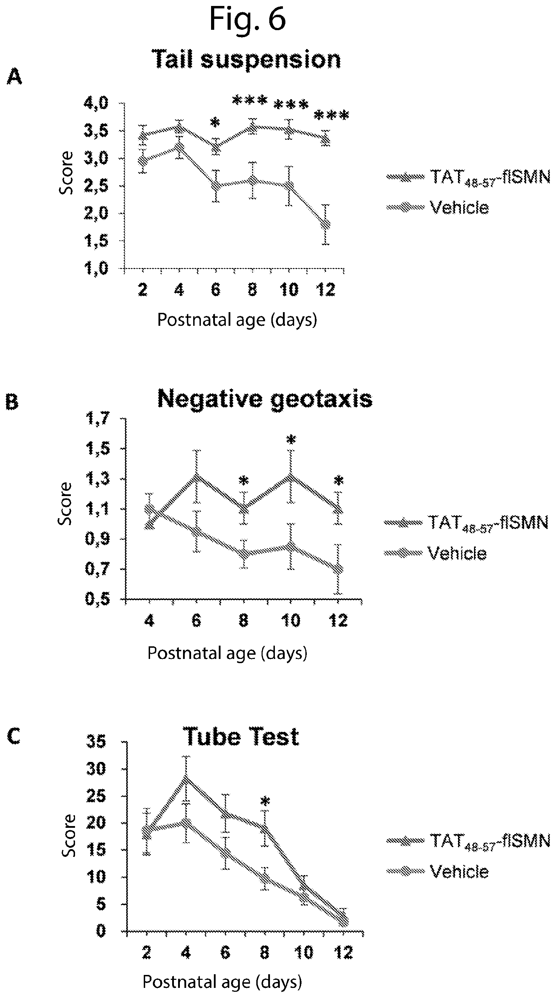

As shown in Figure 6, the protocol of administration used induced a statistically greater improvement in performance on the Tail Suspension test at all time-points analysed. Furthermore, the scores registered on the Negative Geotaxis and Tube tests resulted always better for TAT₄₈₋₅₇-flSMN-treated mice when compared to vehicle.

In Figure 6, data are expressed as mean ± s.e.m.*p<0.05; ***p<0.001 vs vehicle, two-way ANOVA repeated measures, followed by Bonferroni post hoc test for Tail Suspension; *p<0.05 vs vehicle, unpaired t-test for Negative Geotaxis and Tube Tests.

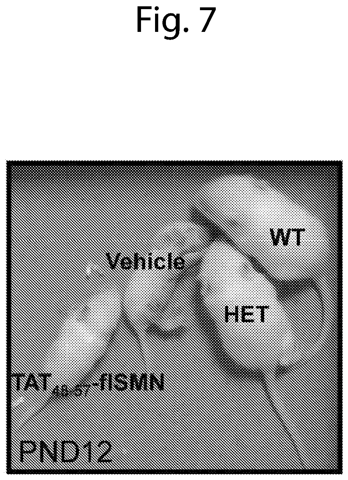

Figure 7 shows the appearance of a TAT₄₈₋₅₇-flSMN-treated SMN*Δ*7 mouse, a vehicle-treated SMN*Δ*7 mouse, a healthy wild-type (WT) mouse and a non-symptomatic heterozygous (HET) mouse at PND12. The healthy wild-type (WT) mouse and the non-symptomatic heterozygous (HET) mouse had not undergone any treatment. Similar to WT and HET mice, the TAT₄₈₋₅₇-flSMN-treated SMN*Δ*7 mouse can stand on its feet, whereas the vehicle-treated SMN*Δ*7 mouse cannot.

Lastly, the treatment with the TAT₄₈₋₅₇-flSMN protein induced a statistically significant improvement in the mean survival rate (vehicle-treated mice (n=20) = 13.25 ± 0.94 days; TAT₄₈₋₅₇-flSMN-treated mice (n=19) = 15.68 ± 0.47 days; *p<0.05 vs. vehicle, unpaired t-test; see Table 1).

**Table 1 - Mean survival in SMNΔ7 mice treated with vehicle (n=20 mice) or TAT₄₈₋₅₇-flSMN (17 mg/kg i.v. at PND1 and i.p. at PND5, n=19 mice). Values are mean ± s.e.m.; *p<0.05 vs vehicle, unpaired t-test.**

| Survival | |
|---|---|
| Vehicle | TAT₄₈₋₅₇- flSMN |
| 13.25 ± 0.94 | 15.68 ± 0.47 |
| n=20 | n=19 |

| | |
|---|---|
| n = population size | |

## Claims

1. A fusion peptide containing a carrier portion and an active peptide portion, said fusion peptide consisting of:
- a carrier peptide portion of SEQ ID NO: 1;
- an active peptide portion of SEQ ID NO: 28 or SEQ ID NO: 29; and
- optionally a linking peptide sequence.

2. Fusion peptide according to claim 1, wherein said linking peptide sequence consists of the amino acid residues -GG-.

3. A fusion peptide as defined in any one of claims 1 or 2 for use as medicament.

4. A fusion peptide as defined in any one of claims 1 or 2 for use in the prevention, symptom postponement or treatment of Spinal Muscular Atrophy (SMA).

5. A combination of two fusion peptides, which comprises:
- a first fusion peptide containing a carrier portion and an active peptide portion, said fusion peptide consisting of:
a) a carrier peptide portion of SEQ ID NO: 1;
b) an active peptide portion of SEQ ID NO: 28; and optionally a linking peptide sequence; and
- a second fusion peptide containing a carrier portion and an active peptide portion, said fusion peptide consisting of:
c) a carrier peptide portion of SEQ ID NO: 1;
d) an active peptide portion of SEQ ID NO: 29 and optionally a linking peptide sequence.

6. A combination as defined in claim 5 for use as medicament.

7. A combination as defined in claim 5 for use in the prevention, symptom postponement or treatment of Spinal Muscular Atrophy (SMA).

8. A pharmaceutical composition comprising the fusion peptide as defined in any one of claims 1 or 2 or the combination of two fusion peptides as defined in claim 5.

9. A pharmaceutical composition as defined in claim 8 for use in the prevention, symptom postponement or treatment of Spinal Muscular Atrophy (SMA).

## Patentansprüche

1. Fusionspeptid, das einen Trägeranteil und einen aktiven Peptidanteil enthält, wobei das Fusionspeptid besteht aus:
- einem Trägerpeptidanteil von SEQ ID NO: 1,
- einem aktiven Peptidanteil von SEQ ID NO: 28 oder SEQ ID NO: 29; und
- optional einer verbindenden Peptidsequenz.

2. Fusionspeptid nach Anspruch 1, wobei die verbindende Peptidsequenz aus den Aminosäureresten -GG- besteht.

3. Fusionspeptid nach einem der Ansprüche 1 oder 2 zur Verwendung als Arzneimittel.

4. Fusionspeptid nach einem der Ansprüche 1 oder 2 zur Verwendung bei der Vorbeugung, Verzögerung des Auftretens von Symptomen oder Behandlung von spinaler Muskelatrophie (SMA).

5. Kombination aus zwei Fusionspeptiden, die Folgendes umfasst:
- ein erstes Fusionspeptid, das einen Trägeranteil und einen aktiven Peptidanteil enthält, wobei das Fusionspeptid besteht aus:
a) einem Trägerpeptidanteil von SEQ ID NO: 1;
b) einem aktiven Peptidanteil von SEQ ID NO: 28; und optional einer verbindenden Peptidsequenz; und
- einem zweiten Fusionspeptid, das einen Trägeranteil und einen aktiven Peptidanteil enthält, wobei das Fusionspeptid besteht aus:
c) einem Trägerpeptidanteil von SEQ ID NO: 1;
d) einem aktiven Peptidanteil von SEQ ID NO: 29 und optional einer verbindenden Peptidsequenz.

6. Kombination nach Anspruch 5 zur Verwendung als Arzneimittel.

7. Kombination nach Anspruch 5 zur Verwendung bei der Vorbeugung, Verzögerung des Auftretens von Symptomen oder Behandlung von spinaler Muskelatrophie (SMA).

8. Pharmazeutische Zusammensetzung, umfassend das Fusionspeptid nach einem der Ansprüche 1 oder 2 oder die Kombination aus zwei Fusionspeptiden nach Anspruch 5.

9. Pharmazeutische Zusammensetzung nach Anspruch 8 zur Verwendung bei der Vorbeugung, der das Auftreten von Symptomen verzögernden Behandlung von spinaler Muskelatrophie (SMA).

## Revendications

1. Peptide de fusion contenant une partie porteuse et une partie peptidique active, ledit peptide de fusion étant constitué de:
- une partie peptidique porteuse de SEQ ID NO: 1,
- une partie peptidique active de SEQ ID NO: 28 ou SEQ ID NO: 29; et
- éventuellement une séquence peptidique de liaison.

2. Peptide de fusion selon la revendication 1, dans lequel ladite séquence peptidique de liaison est constituée de l'acide aminé résidus -GG-.

3. Peptide de fusion tel que défini dans l'une quelconque des revendications 1 ou 2 destiné à être utilisé comme médicament.

4. Peptide de fusion tel que défini dans l'une quelconque des revendications 1 ou 2 destiné à être utilisé dans la prévention, le report des symptômes ou le traitement de l'amyotrophie spinale (SMA).

5. Combinaison de deux peptides de fusion, qui comprend:
- un premier peptide de fusion contenant une partie porteuse et une partie peptidique active, ledit peptide de fusion étant constitué de:
a) une partie peptidique porteuse de SEQ ID NO: 1;
b) une partie peptidique active de SEQ ID NO: 28; et éventuellement une séquence peptidique de liaison; et
- un deuxième peptide de fusion contenant une partie porteuse et une partie peptidique active, ledit peptide de fusion étant constitué de:
c) une partie peptidique porteuse de SEQ ID NO: 1;
d) une partie peptidique active de SEQ ID NO: 29 et éventuellement une séquence peptidique de liaison.

6. Combinaison telle que définie dans la revendication 5 destinée à être utilisée comme médicament.

7. Combinaison telle que définie dans la revendication 5 destinée à être utilisée dans la prévention, le report des symptômes ou le traitement de l'amyotrophie spinale (SMA).

8. Composition pharmaceutique comprenant le peptide de fusion tel que défini dans l'une quelconque des revendications 1 ou 2 ou la combinaison de deux peptides de fusion telle que définie dans la revendication 5.

9. Composition pharmaceutique telle que définie dans la revendication 8 destinée à être utilisée dans la prévention, le report des symptômes, le traitement de l'amyotrophie spinale (SMA).
